# EUROPEAN PATENT SPECIFICATION

(11) **EP 4 003 384 B1**
(45) Date of publication and mention of the grant of the patent: **25.06.2025**
(21) Application number: 20850736.8
(22) Date of filing: 05.08.2020
(51) Int. Cl.: A61K 36/185, A23L 33/105, A61K 9/107

(54) **AN EXTRACT FROM CANNABIS AND PEGANUM**
EXTRAKT AUS CANNABIS UND PEGANUM
EXTRAIT DE CANNABIS ET DE PEGANUM

(30) Priority: 07.08.2019 TR 201912068
(43) Date of publication of application: 01.06.2022
(73) Proprietor: T.C. Erciyes Universitesi, 38039 Kayseri (TR)
(72) Inventor: ÇOBAN, Abdullah, Melihgazi/Kayseri (TR); BENK, Ayse, Kocasinan/Kayseri (TR); SEYFELI, Canan Selcen, Kayseri (TR)
(74) Representative: Yamankaradeniz, Kemal
(86) International application number: PCT/TR2020/050681
(87) International publication number: WO 2021/025652

(56) References cited:
- WO-A1-2017/145160
- IL-A- 261 774
- US-A1- 2018 153 948
- US-B1- 10 308 625
- NIROUMAND MINA CHERAGHI ET AL: "Medicinal properties of Peganum harmala L. in traditional Iranian medicine and modern phytotherapy: a review", JOURNAL OF TRADITIONAL CHINESE MADICINE- CHUNG I TSA CHIH YING WEN PAN, ELSEVIER, AMSTERDAM, NL, vol. 35, no. 1, 11 May 2015 (2015-05-11), pages 104 - 109, XP029575039, ISSN: 0254-6272, DOI: 10.1016/S0254-6272(15)30016-9
- PATTNAIK FALGUNI ET AL: "Cannabis: Chemistry, extraction and therapeutic applications", CHEMOSPHERE, PERGAMON PRESS, OXFORD, GB, vol. 289, 25 November 2021 (2021-11-25), XP086911749, ISSN: 0045-6535, [retrieved on 20211125], DOI: 10.1016/J.CHEMOSPHERE.2021.133012
- KARTAL M ET AL: "HPLC method for the analysis of harmol, harmalol, harmine and harmaline in the seeds of Peganum harmala L.", JOURNAL OF PHARMACEUTICAL AND BIOMEDICAL ANALYSIS, vol. 31, no. 2, 1 February 2003 (2003-02-01), AMSTERDAM, NL, pages 263 - 269, XP093125024, ISSN: 0731-7085, Retrieved from the Internet <URL:https://pdf.sciencedirectassets.com/271442/1-s2.0-S0731708500X02063/1-s2.0-S073170850200568X/main.pdf?X-Amz-Security-Token=IQoJb3JpZ2luX2VjEDgaCXVzLWVhc3QtMSJHMEUCIAdRDN1Y968Qv6/1CdUGinSSPYTdzDg6NkeFP0jPm2OqAiEA9vadJqkT6N+KcJfNQFC2OfnZC87euGOqXmj+4KnOGSgquwUI8P//////////ARAFGgwwNTkwMDM1NDY4NjUiDHgAS> DOI: 10.1016/S0731-7085(02)00568-X
- SIEWERT BIANKA ET AL: "The photoactivity of natural products - An overlooked potential of phytomedicines?", PHYTOMEDICINE, ELSEVIER, AMSTERDAM, NL, vol. 60, 10 June 2019 (2019-06-10), XP085813612, ISSN: 0944-7113, [retrieved on 20190610], DOI: 10.1016/J.PHYMED.2019.152985
- BENZEKRI, R. ET AL.: "Anti HSV-2 activity of Peganum harmala (L.) and isolation of the active compound", MICROBIAL PATHOGENESIS, vol. 114, 2018, pages 291 - 298, XP085349484, DOI: 10.1016/j.micpath.2017.12.017

## Description

### Field of the Invention

The present invention relates to a plant extract from cannabis and *peganum* produced by cold extraction and a method for producing this extract.

Specifically, the invention relates to the fields of use of the invention.

### State of the Art

Today, plants have a wide variety of uses in human welfare and wellbeing, including cosmetics, perfumery, pharmaceutical and food industries. The number of experiments and research on plants has increased by scientist as the various benefits of plants have been seen in all areas.

As two of the oldest known plant raw materials in human history, cannabis and *peganum harmala* are being utilized in most of the industries described above and research continues into the benefits and applications of these materials. Cannabis is an annual plant genus in the family Cannabaceae. It is the most well - known agricultural plant in the family as one of the oldest plant raw materials in human history. The fibres in the stem can be used in yarn, weaving and cloth production, while the pulp section is utilized for paper production. The plant is also being used for medicinal purposes, in cosmetics and other industries to produce a broad range of products. Its Latin name is cannabis. Morphologically, cannabis has three basic species:
- cannabis sativa
- cannabis indica
- cannabis ruderalis

Cannabis Sativa: The plant can grow up to 3 meters high. The flowering time takes 9 to 12 weeks. It has long and thin leaves. Contains high amount of a psychoactive constituent known as Tetrahydrocannabinol (THC).

Cannabis Indica: The plant is shorter compared to sativa species. Flowering time is 7 to 9 weeks. The plant has wide and short leaves. It is more densely branched and thus woodier. Contains high amount of cannabidiol (CBD), which is a non - psychoactive substance.

Cannabis Ruderalis: The plant has a shorter stature with shorter flowering time compared to other species. Looks more like a bush with small leaves. Contains very low amount of Tetrahydrocannabinol (THC).

Cannabis contains biologically active constituents and more than 500 different chemical compounds. It includes primary metabolites like amino acids, fatty acids and steroids as well as secondary metabolites including cannabinoids, flavonoids, stilbenoids, terpenoids, lignans and alkaloids. The concentration of compounds varies depending on the type, age, cultivation conditions (humidity, light, fertilization, etc.) time of harvest and storage conditions.

One of the compound groups in the plant, cannabinoids are pharmacologically active and hence most research has been conducted on this group and the classification of the plant takes place according to the amount of cannabinoid contained. More than 60 types of cannabinoids are contained within the plant, but only a few of them have been studied in detail and the biological activities of the remaining types have not been adequately researched. The full pharmacological activity of the plant has been explained through these compounds and the effects of other compound groups have not been taken into consideration.

Cannabinoids can be classified into three groups:
- Phytocannabinoids
- Endocannabinoids
- Synthetic cannabinoids

Phytocannabinoids: Naturally produced in the plant. The classical cannabinoids are derived from their respective 2 - carboyxlic aids by decarboxylation. This reaction is catalysed by heat, light or alkaline conditions. Phytocannabinoids are not soluble in water but are soluble in alcohols, lipids and other non - polar organic solvents. Water - soluble phenolate salts are derived in alkaline conditions. There are 66 known phytocannabinoids in the cannabis plant. Delta - 9 - tetrahydrocannabinol (Δ9 - THC) is the most researched primary psychoactive component of the cannabis plant. Other well - known cannabinoids include cannabidiol (CBD) and cannabinol (CBN).

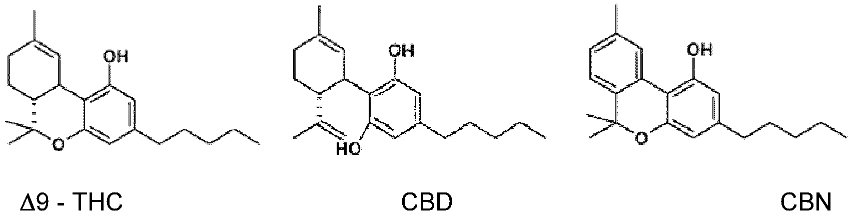

Endocannabinoids: Endocannabinoids are produced within human and animal bodies and are defined as endogenous ligands of cannabinoids receptors (CB1 and CB2). Its chemical structure is made up of lipophilic molecules from arachidonic acids. The two known endocannabinoids are arachidonyl ethanolamine (anandamide, AEA) and 2 - arachidonylgylcerol (2 - AG). Endocannabinoid system is known to have growing evidence regarding in its role in regulation of various physiological conditions and diseases. *(*The Pharmacology of Cannabinoids, Prof. Dr. Ahmet Ulugöl, Trakya University, Faculty of Medicine, Department of Medicinal Pharmacology) (The Endocannabinoidome The World of Endocannabinoids and Related Mediators 2015, pages 137 - 152*)*

Synthetic Cannabinoids: Synthesized in laboratories in ways similar to natural cannabinoids and particularly the psychoactive Tetrahydrocannabinol (THC). Unlike Tetrahydrocannabinol (THC), it has higher affinity to cannabinoid receptors and is thus more poisonous and dangerous. Use of synthetic cannabinoids (agitation, anxiety, etc.) can lead to psychiatric (agitation, anxiety, etc.), cardiovascular (hypertension, myocardial infarction, etc.), neurological and gastronomical diseases. *(*CMAJ, February 18, 2014, 186(3) Synthetic Cannabinoids*)*

Almost all existing knowledge on the pharmacological effects and results of cannabinoids are based on Tetrahydrocannabinol (THC). There is not enough information in the literature related to the effect mechanisms of other cannabinoids and other compound groups.

The second plant used in the present invention is *peganum. Peganum harmala* is the species grown and used in the country. **It** is a perennial, hairless plant growing to 60 - 70 cm. The alkaloids contained particularly in the seeds and roots are the active components of the plant. The main alkaloids found in the plant are harmine, harmaline, harmol, harmalol and harmane. The herbal drugs derived from the seeds are known to have hypothermic, antibacterial, antifungal, antiviral, antitumor and analgesic properties.

Literature review on the field of the invention reveals the following patent applications. The application numbered US6403126B1 describes a method of extracting cannabinoids, cannflavins and / or essential oils from hemp. According to this method, first industrial hemp is harvested, and the chaff is threshed from the seeds. The chaff is then ground, and the ground chaff is extracted with an organic solvent. Petroleum derived hydrocarbons, toluene, trimethylpentane; small molecular weight alcohols (ethyl alcohol), hydrocarbons containing chlorine (chloroform and dichloromethane) are used as solvents. Chromatographic methods are used to fractionate the specific compounds in the extract. Cannflavins, essential oils, D9 - THC and cannabinoids can be separated by adjusting chromatographic conditions. However, this application does not use other sections of the plant with high concentrations of the mentioned active substances.

Another invention is the PCT application numbered WO2016064987A1, titled "Cannabis extracts and methods of preparing and using same". The invention relates to the extraction of pharmaceutically active compounds from cannabis and preparation of a botanical drug substance. Cannabis leaves and seeds obtained from the plant are stored in cold conditions. The plant material is removed from the freezer, spread into small - sized pieces, then supercritical carbon dioxide extraction method is applied. The result material is winterized, then filtered before getting ready for use. The product can be turned into a botanical drug substance with further additives. However, the supercritical carbon dioxide extraction mentioned in this application is an expensive method that is not common. Moreover, it is also clear that pharmacologically active compounds can decompose and change forms as a result of side reactions with carbon dioxide.

Another related invention is the national application numbered 2018/00236 with the title "Herbal Mixture to Remove Diabetes and the Method of Producing the Same". The invention relates to an herbal mixture to permanently remove the diabetes, which is a serious disease caused by insulin deficiency or ineffectiveness and the method of preparing the same. The invention makes use of *peganum harmala* powder and horse chestnut powder. The mixture prepared in the invention is intended for use only in curing diabetes.

Due to the disadvantages of the above inventions and the limitations of the existing methods, there is a clear need for improvement in the technical field of the invention.

### Summary of the Invention

The present invention relates to a plant extract from cannabis and *peganum* produced by cold extraction that meets the requirements described above, eliminates all disadvantages and bring some additional benefits.

Specifically, the object of the present invention is to prepare a natural plant extract composed of cannabis, *peganum,* edible vegetable oil (sesame oil, black cumin oil, olive oil, sunflower seed oil, etc.) and edible non - toxic solvents (DMSO, etc.), which has a completely herbal composition and is not harmful to human health compared to synthetic - based formulations used in the prior art.

Another object of the invention, the cannabis and *peganum hermala* plants are used in full to prepare a plant extract that utilizes all active components.

Another object of the invention relates to use of cold extraction method in preparing the mentioned plant extract to prevent decomposition of active components in the plants.

One other object of the invention is to boost the immune system by way of the extract to help fight against several diseases.

Another object of the invention is to enable the product to be used in early diagnosis and operations thanks to its fluorescence properties.

The invention specifically aims to present a plant extract with natural ingredients which will be available for use in various fields.

The structural properties and characteristics of the invention will be more clearly understood and therefore, best appreciated by reading the detailed description below.

### Detailed Description of The Invention

The detailed description of the invention is related to the plant extract from cannabis and *peganum hermala* plants produced by cold extraction and intended for purposes of illustrating the embodiments of the invention in a way not limiting the scope of the disclosure.

The present invention relates to a plant extract of cannabis and *peganum hermala* plants which revealed green fluorescence under 320 - 405 nm wavelength of UV light; wherein the plant extract is produced by cold extraction method comprising the following steps.
- Grinding of the leaves, stem, seeds, roots and any other part of the cannabis plant without separating any part of it;
- Grinding of the leaves, stem, seeds, roots and any other part of the *peganum hermala* plant without separating any part of it;
- Mixing hemp seed oil and dimethyl sulfoxide (DMSO) in a ratio of 1:1 by weight to prepare a solvent mixture;
- Putting the ground cannabis and the ground *peganum hermala* into separate containers and mixing them with the prepared solvent mixture in a ratio of 1:1 to 1:5 plant:solvent by weight;
- Freezing of obtained the mixtures separately for 24 hours at a temperature range of - 50 °C to 0 °C preferably at -18 °C;
- Thawing the frozen mixtures separately under room conditions for 24 hours;
- Repeating the freezing and thawing steps one after the other multiple times, preferable 7 times;
- Leaching of thawed mixtures, separetly thus obtaining cannabis and *peganum hermala* extracts;
- Mixing the cannabis and *peganum hermala* extracts in a ratio of 1:1 by weight.

Cannabis is an annual wood - like dicotyledonous plant genus in the family Cannabaceae. The plant can grow from 50 cm up to 3 m. The stem of the plant is upright, but hollow inside and its surface is rough due to thorn - like hair. The plant is dioecious with distinct male and female individuals organisms. Male cannabis pollinates while female cannabis flowers and contains high amounts of Tetrahydrocannabinol. The plant has three main species, namely Cannabis indica, Cannabis sativa and Cannabis ruderalis. Cannabis is known to have health benefits for humans. Some of these benefits can be listed as follows:
- Very rich in amino acids, which boost the functioning of the human body.
- Cannabis seed has a hormonal regulatory effect thanks to rich content of the volatile fatty acid known as Gamma Linolenic Acid.
- The seed oil also supports the health of brain and the formations in the brain. Reduces the risk of forgetfulness, Parkinson's disease and autism.
- When consumed regularly, rejuvenates the dermis of the skin with antiaging properties and promotes a fresh and bright look of the skin. The same benefit also applies to hair and scalp. Protects the DNA structure of the skin, hair and nails thanks to its Vitamin E content.
- The contained magnesium helps increase melatonin production in the brain and reduce sleep problems.

*Peganum harmala* is a plant of the Nitrariaceae family growing in hot regions of Africa, Asia and America. **It** is a ramulose plant with white flowers. **It** grows up to 35 cm in steppes as a perennial herbaceous plant. The flowers are greenish white in colour. The fruit is in the form of round seed capsules. Only seeds of the plant are used. Along with vitamins A and C, the seeds contain glycosides called harmaline, harmine, harmalol, peganine; and a red dye agent. The herbal medicine derived from the seeds are known to have hypothermic, antibacterial, antifungal, antitumor and analgesic properties. *Peganum harmala* has the following health benefits for humans:
- **It** reduces shortness of breath, heart palpitations.
- Good for Parkinson's Disease.
- Good for sleeplessness and regulates sleep.
- Soothes stomach aches.
- Antibacterial properties.

According to the invention, a plant extract is produced from cannabis and *peganum hermala* plants by cold extraction and the method for producing the same includes at least one edible solvent and at least one edible oil for dissolving the plants.

The edible solvent (DMSO) used in the invention is dimethyl sulfoxide (DMSO), an organosulfur compound with the formula (CH₃) 2SO. This colourless liquid is an important polar aprotic solvent and is also being recently used as a pharmacological agent. **It** easily penetrates the skin and leaves a perceived garlic taste. Dimethyl sulfoxide is derived from the Kraft pulp and is a by - product of dimethyl sulphide. DMSO is produced by oxidation of dimethyl sulphide with oxygen or nitrogen dioxide. As a polar solvent, DMSO is less toxic than dimethylformamide, dimethylacetamide, N - methyl - 2 - pyrrolidone, and HMPA. DMSO is used as a solvent salt in chemical reactions, and in particular as a solvent for chemical reactions involving Finkelstein reactions and other nucleophilic substitutions.

Edible oils should be selected from a group of edible oils not harmful to human health. Sesame oil, black cumin oil, olive oil, sunflower seed oil or combinations thereof can be used for this purpose. According to the preferred embodiment of the invention, hemp oil derived from hemp seed is used as the edible oil.

**In** light of the information and benefits described above, the object of the invention is to develop a method for extracting all components of cannabis, which contains a psychoactive substance known as THC that has been the subject of hundreds of studies, according to their natural proportions without separating certain parts of the plant (flower, leaf, stem, etc.), independent of the species of the plant (industrial or medicinal), in a directly edible form without applying any heat treatment that may decompose biologically active components. The end product will be readily usable internally and externally in health applications. The product will have a fixed concentration of active substances and composition, thereby further facilitating the use.

Fruits, vegetables and other plants should be used and consumed in full to produce health benefits. Because interactions of various biologically active substances between themselves and / or with different receptors in the body make them more health - effective and nutritious. Extracting only one or several of these active substances falls short in bringing the full effect of the substances. This is because the plant contains the ideal amount of active and inactive compounds. *(*Christelle M. Andre, Jean - Francois Hausman, Gea Guerriero, Cannabis sativa: The Plant of the Thousand and One Molecules, Front Plant Sci. 2016; 7: 19*).*

An embodiment of the invention is carried out according to the study detailed below:

### Study

Materials: The cannabis plant used in the works is obtained from the authorized producer based in Kastamonu. The *peganum hermala* seeds is supplied from the herbalists. Edible oils and edible solvents (DMSO) were used in the solvent mixture for the extractions. All edible oils including sesame oil, black cumin oil, olive oil, sunflower seed oil, and particularly hemp seed oil is preferable for the invention. Dimethyl sulfoxide is a commercial solvent derived from paper and has been used in recent years particularly as a pharmacological agent. Since it is soluble both in organic and aqueous solutions, it is an effective solvent for water - insoluble compounds. Due to this property, it is used as carrier of biologically active compounds. The solvent is easily removed from the body when applied internally or externally and has no damaging effects on the cell or cell membrane. It is also known as an effective antioxidant due to its ability to neutralize free radicals. Research revealed that DMSO is an effective carrier which can easily penetrate very - hard - to - penetrate regions like bones and cerebral cortex and transmit active components only to regions with disease. Therefore, DMSO was preferred in the study to facilitate extraction and transmission of active components from the plant to regions with disease.

### Methodology

Cannabis Extraction: 50 g of medical cannabis collected, dried and stored under suitable conditions was ground in full without separating any part of it (stem, fibre, etc.). 75 g edible oil (hemp seed oil) and 75 g edible solvent (DMSO) was added to the ground cannabis. The solvent solution contains edible oil / edible solvent (DMSO) in the ratio of 1:1 by weight, and cannabis / solvent ratio should be 1:1 to 1:5 by weight. Solvent mixture was mixed with cannabis for 5 minutes to ensure thorough distribution. The mixture was added to a glass jar with lid and placed in deep freezer. It was taken out after 24 hours in the freezer at a temperature range between 0 °C and -50 °C, and preferably at -18 °C. The external surface of the jar was covered so that it does not receive sunlight and is kept under room conditions for 24 hours. After repeating this process 7 times, the extract was filtered through a filter paper. The end product was stored in a glass bottle with lid.

*Peganum Hermala* Extraction: Same steps with cannabis extraction were followed with the same proportions and methods.

### Analysis

Cannabinoids in cannabis and alkaloids in *peganum hermala* have fluorescence properties. *(*Arno Hazekamp, Anja Peltenburg, Rob Verpoorte & Christian Giroud (2005) Chromatographic and Spectroscopic Data of Cannabinoids from Cannabis sativa L., Journal of Liquid Chromatography & Related Technologies, 28:15, 2361 - 2382, DOI: 10.1080 / 10826070500187558*) (*Bahram Hemmateenejada,*, Mojtaba Shamsipur b, Fayezeh Samari a, Taghi Khayamianc, Malihe Ebrahimi c, Zahra Rezaei, Combined fluorescence spectroscopy and molecular modeling studies on the interaction between harmalol and human serum albumin, Journal of Pharmaceutical and Biomedical Analysis 67-68 (2012) 201-208*)*

The extract was put into a test tube to check the fluorescence properties with UV light. UV light at 320 - 405 nm wavelength revealed green fluorescence in the sample. The light was in visible area and was visible to the eye with no filter. Filters transmitting varying wavelengths can be used for different analyses.

### Application

Extracts derived from cannabis and *peganum hermala* were mixed at 1:1 ratio by weight, and a drop was applied to skin surface. No difference was observed on the skin under normal lighting conditions, but green fluorescence was visible under UV 405 nm light conditions. Despite application on a very limited skin surface, it was observed that extracts with fluorescence properties were absorbed and spread in short time in all directions across veins and capillary vessels.

### Usage

Extracts derived from cannabis and *peganum hermala* can be used particularly in diagnosis and treatment of cancer. Separately produced cannabis and *peganum hermala* extracts can be mixed as desired in amounts between 1 to 99 % before use. The mixture can be fed into the body with a serum or can be directly used in intravenous application. Moreover, it is also suitable for direct oral application as it is easily removable from the body through the urinary system. Compounds extracted from the plants are effective against cancer with fluorescence properties particularly thanks to the carrier property of Dimethyl Sulfoxide (DMSO) and are directly transmitted to cancer cells. This can be implemented as a treatment method, while it will also be possible to see very small cancer cells not identifiable with existing techniques through the fluorescence characteristics of cancer cells particularly in early diagnosis and operations. **In** this manner, the surgeon will easily see the cancerous region and perform the operation without missing any cancer cells. Moreover, it will be possible to monitor the effect mechanisms of active substances in cancer cells through the same method.

The extract can be used particularly for the purpose of cancer. **In** addition, the extract can also be used in itch, eczema, pimple, fungus, acne treatment and cosmetic applications.

Moreover, the extract can also be used as dietary supplement to boost immune system.

Apart from these fields of application, the extract can also be used as an insecticide, biocide, fungicide, herbicide and antimicrobial agent in the form of emulsion and micro emulsions. Due to high antimicrobial activity, the extract can easily be used for all disinfection purposes including body disinfection. Moreover, it can be easily mixed with essential oils (tea tree oil, peppermint oil, eucalyptus oil, cinnamon oil, thyme oil, d - limonene, etc.) in emulsion and micro emulsion forms and various compositions can be prepared depending on the intended use. Additionally, emulsion and micro emulsion forms can be diluted with water for use in protection and prevention of degradation of agricultural products like vegetables and plants. The diluted form of the product can also be easily used in treatment of many conditions including hair loss and dandruff. Due to its fluorescence capabilities, the extract can also be used in various fields where confidential marking is performed (banknotes, secure barcodes, etc.)

## Claims

1. A plant extract of *Cannabis* and *Peganum hermala* plants which revealed green fluorescence under 320 - 405 nm wavelength of UV light; wherein the plant extract is produced by a cold extraction method comprising the following steps:
i. Grinding of leaves, stems, seeds, roots and any other part of the *Cannabis* plant without separating any part of it;
ii. Grinding of the leaves, stems, seeds, roots and any other part of the *Peganum hermala* plant without separating any part of it;
iii. Mixing hemp seed oil and dimethyl sulfoxide (DMSO) in a ratio of 1:1 by weight to prepare a solvent mixture;
iv. Putting the ground *Cannabis* and the ground *Peganum hermala* into separate containers and mixing them with the prepared solvent mixture in a ratio of 1:1 to 1:5 plant:solvent by weight;
v. Freezing of obtained the mixtures separately for 24 hours at a temperature range of - 50 °C to 0 °C preferably at -18 °C;
vi. Thawing the frozen mixtures separately under room conditions for 24 hours;
vii. Repeating the steps v and vi one after the other multiple times, preferable 7 times;
viii. Leaching of thawed mixtures separately thus obtaining *Cannabis* and *Peganum hermala* extracts;
ix. Mixing the *Cannabis* and *Peganum hermala* extracts in a ratio of 1:1 by weight.

2. The plant extract according to Claim 1, **characterized in that**; the said cannabis is either cannabis sativa, cannabis indica, cannabis ruderalis and / or a hybrid thereof.

3. The plant extract according to Claim 1, wherein the said extract is intended for use as a fluorescent agent applied to the skin.

## Patentansprüche

1. Pflanzenextrakt von *Cannabis-* und *Peganum-hermala-Pflanzen,* der unter einer Wellenlänge von 320 - 405 nm von UV-Licht grüne Fluoreszenz zeigte; wobei der Pflanzenextrakt durch ein Kälteextraktionsverfahren hergestellt wird, das die folgenden Schritte umfasst:
i. Vermahlen von Blättern, Stängeln, Samen, Wurzeln und jedes anderen Teils der *Cannabis-Pflanze,* ohne einen Teil davon zu trennen;
ii. Vermahlen der Blätter, Stängel, Samen, Wurzeln und jedes anderen Teils der *Peganum-hermala-Pflanze,* ohne einen Teil davon zu trennen;
iii. Mischen von Hanfsamenöl und Dimethylsulfoxid (DMSO) in einem Gewichtsverhältnis von 1:1, um ein Lösungsmittelgemisch herzustellen;
iv. Füllen des gemahlenen *Cannabis* und des gemahlenen *Peganum hermala* in getrennte Behälter und Mischen dieser mit dem hergestellten Lösungsmittelgemisch in einem Gewichtsverhältnis von 1:1 bis 1:5 Pflanze:Lösungsmittel;
v. Einfrieren der erhaltenen Gemische getrennt für 24 Stunden bei einem Temperaturbereich von -50 °C bis 0 °C, vorzugsweise bei -18 °C;
vi. Auftauen der gefrorenen Gemische getrennt unter Raumbedingungen über 24 Stunden;
vii. Wiederholen der Schritte v und vi mehrere Male nacheinander, vorzugsweise 7-mal;
viii. Separates Auslaugen der aufgetauten Gemische, wodurch *Cannabis-* und *Peganum-hermala-Extrakte* erhalten werden;
ix. Mischen der Extrakte von *Cannabis* und *Peganum hermala* in einem Gewichtsverhältnis von 1:1.

2. Pflanzenextrakt nach Anspruch 1, **dadurch gekennzeichnet, dass**; das Cannabis entweder Cannabis sativa, Cannabis indica, Cannabis ruderalis und / oder ein Hybrid davon ist.

3. Pflanzenextrakt nach Anspruch 1, wobei der Extrakt zur Verwendung als ein fluoreszierendes Mittel bestimmt ist, das auf die Haut aufgetragen wird.

## Revendications

1. Extrait végétal de plantes *Cannabis* et *Peganum hermala* qui a révélé une fluorescence verte sous une longueur d'onde de 320 à 405 nm de lumière UV ; ledit extrait végétal étant produit par un procédé d'extraction à froid comprenant les étapes suivantes :
i. le broyage des feuilles, tiges, graines, racines et toute autre partie de la plante *Cannabis* sans en séparer aucune partie ;
ii. le broyage des feuilles, tiges, graines, racines et toute autre partie de la plante *Peganum hermala* sans en séparer aucune partie ;
iii. le mélange d'huile de graines de chanvre et de diméthylsulfoxyde (DMSO) dans un rapport de 1:1 en poids pour préparer un mélange de solvants ;
iv. le placement du *Cannabis* broyé et du *Peganum hermala* broyé dans des récipients séparés et leur mélange avec le mélange de solvants préparé dans un rapport de 1:1 à 1:5 plante:solvant en poids ;
v. la congélation des mélanges obtenus séparément pendant 24 heures à une plage de température de -50 °C à 0 °C, de préférence à -18 °C ;
vi. la décongélation des mélanges congelés séparément dans des conditions ambiantes pendant 24 heures ;
vii. la répétition des étapes v et vi l'une après l'autre plusieurs fois, de préférence 7 fois ;
viii. la lixiviation des mélanges décongelés séparément, obtenant ainsi des extraits de *Cannabis* et de *Peganum hermala ;*
ix. le mélange des extraits de *Cannabis* et de *Peganum hermala* dans un rapport de 1:1 en poids.

2. Extrait végétal selon la revendication 1, **caractérisé en ce que** ; ledit cannabis est soit du cannabis sativa, du cannabis indica, du cannabis ruderalis et/ou un hybride de ceux-ci.

3. Extrait végétal selon la revendication 1, ledit extrait étant destiné à être utilisé en tant qu'agent fluorescent appliqué sur la peau.
